# EUROPEAN PATENT SPECIFICATION

(11) **EP 3 411 021 B1**
(45) Date of publication and mention of the grant of the patent: **14.05.2025**
(21) Application number: 17703477.4
(22) Date of filing: 02.02.2017
(51) Int. Cl.: A61K 9/20, A61K 9/28, A61K 9/48, A61K 31/216, A61K 31/225, A61K 31/41, A61K 45/06, A61P 9/00

(54) **NEW USE OF A COMBINATION OF SACUBITRIL AND VALSARTAN**
NEUE VERWENDUNG EINER KOMBINATION VON SACUBITRIL UND VALSARTAN
NOUVELLE UTILISATION D'UNE COMBINAISON DE SACUBITRIL ET DE VALSARTAN

(30) Priority: 03.02.2016 EP 16154153; 09.02.2016 US 201662293005 P; 12.09.2016 US 201662393163 P
(43) Date of publication of application: 12.12.2018
(73) Proprietor: Novartis AG, 4056 Basel (CH)
(72) Inventor: CHEN, Fabian, East Hanover, New Jersey 07936 (US); AYALASOMAYAJULA, Surya Prakash, East Hanover, New Jersey 07936 (US); BUSH, Christopher, East Hanover, New Jersey 07936 (US); BERKHIN, Masha, East Hanover, New Jersey 07936 (US); WINZENBURG, Gesine, 4002 Basel (CH); TRUEBY, Bernd, 4002 Basel (CH)
(74) Representative: Carpmaels & Ransford LLP
(86) International application number: PCT/IB2017/050573
(87) International publication number: WO 2017/134600

(56) References cited:
- WO-A1-2009/061713
- KIRK RICHARD ET AL: "The International Society of Heart and Lung Transplantation Guidelines for the management of pediatric heart failure: Executive summary", JOURNAL OF HEART AND LUNG TRANSPLANTATION, ELSEVIER, AMSTERDAM, NL, vol. 33, no. 9, 17 June 2014 (2014-06-17), pages 888 - 909, XP029042744, ISSN: 1053-2498, DOI: 10.1016/J.HEALUN.2014.06.002
- ANONYMOUS: "European Medicines Agency: Assessment report Entresto International non-proprietary name: SACUBITRIL / VALSARTAN", 24 September 2015 (2015-09-24), pages 1 - 115, XP055609648, Retrieved from the Internet <URL:https://www.ema.europa.eu/en/documents/assessment-report/entresto-epar-public-assessment-report_en.pdf> [retrieved on 20190730]
- ANONYMOUS: "Novartis Entresto receives positive CHMP opinion for pediatric heart failure", 31 March 2023 (2023-03-31), pages 1 - 6, XP093257021, Retrieved from the Internet <URL:https://www.novartis.com/news/media-releases/novartis-entresto-receives-positive-chmp-opinion-pediatric-heart-failure> [retrieved on 20250207]
- ANONYMOUS: "Annex I - Summary of Product Characteristics", 1 January 2023 (2023-01-01), pages 1 - 93, XP093257022, Retrieved from the Internet <URL:https://www.ema.europa.eu/en/documents/product-information/entresto-epar-product-information_en.pdf> [retrieved on 20250307]
- ANONYMOUS: "Commission Implementing Decision", 26 May 2023 (2023-05-26), pages 1 - 4, XP093257023, Retrieved from the Internet <URL:https://ec.europa.eu/health/documents/community-register/2023/20230526159257/dec_159257_en.pdf> [retrieved on 20250307]
- ANONYMOUS: "European Medicines Agency decision P/0106/2014", 11 June 2014 (2014-06-11), pages 1 - 8, XP055286995, Retrieved from the Internet <URL:http://www.ema.europa.eu/docs/en_GB/document_library/PIP_decision/WC500168118.pdf> [retrieved on 20160708]

## Description

The present invention relates to pharmaceutical compositions for use in treating heart failure in a pediatric human patient comprising administration to said patient of a therapeutically effective amount or a prophylactically effective amount of a combination of a therapeutic agent blocking the angiotensin receptor and a therapeutic agent inhibiting the NEP enzyme, in particular of a combination of sacubitril and valsartan in a pharmaceutically acceptable form and in a 1:1 molar ratio.

### Background of the Invention

### Heart Failure in Pediatric Patients

Pediatric heart failure (HF) is characterized by significant morbidity and mortality, frequent hospitalization and medical care, and poor quality of life. It is estimated that between 12,000 to 35,000 children below age 19 are diagnosed with HF in the United States (US) each year. HF can develop or exacerbate in childhood, during adolescence and also later in adulthood as made evident by the growing number of adults with congenital heart disease. Congenital heart disease and cardiomyopathy are the two most common causes of pediatric HF.

The largest HF burden comes from children born with congenital malformations.

Congenital heart disease occurs in approximately 8 per 1,000 live births of which 1-2 per 1,000 develop HF. A wide variety of congenital abnormalities may be present including ventricular or atrial septal defect, patent ductus arteriosus, persistent aorto-pulmonary connection, hypoplastic left heart, aortic or pulmonary vein stenosis, anomalous origin of the left coronary artery, and coarctation of the aorta. Most of these children are diagnosed before ages 1 and many have early surgical intervention, usually before age 2.

The other main cause of pediatric HF is cardiomyopathy, with an estimated annual incidence of 1 per 100,000 children in the US, Australia, United Kingdom and Ireland. Dilated cardiomyopathy (usually diagnosed as idiopathic, familial, or myocarditic) is the most common type. Hypertrophic cardiomyopathy due to familial isolated cardiomyopathy, an inborn error of metabolism, or a malformation syndrome is the next most common type. Cardiomyopathy can also be associated with muscular dystrophies such as Duchenne's muscular dystrophy and myotonic dystrophy. In developing countries, rheumatic heart disease, nutritional deficiencies, and other tropical diseases such as Chagas disease can also be a substantial cause of pediatric HF.

The clinical course and outcome for pediatric HF depends on the etiology. For congenital heart disease, corrective surgery will have a major impact on the clinical course. Following congenital heart surgery, HF can still develop for a number of reasons including myocardial systolic dysfunction.

Many pediatric patients with severe HF are usually listed for heart transplant if available; however, cardiac transplantation is usually a last resort given the limited availability of donor organs, complicated clinical course management and associated morbidity and mortality. Inthe US, one in four infants listed for heart transplant dies before a donor heart is available. Furthermore, nearly 40% of children in the US with symptomatic cardiomyopathy either undergo heart transplantation or die within 2 years.

In contrast to HF in adults, there is very limited research in pediatric HF. Consequently, treatment of HF in children is based on information and results provided by adult studies. Pediatric HF treatment is outlined in the recent guidelines published by the International Society of Heart and Lung Transplantation (ISHLT) (Kirk R, Dipchand AI, Rosenthal DN, et al. (2014) The international society for heart and lung transplantation guidelines for the management of pediatric heart failure: Executive summary. J Heart Lung Transplant; 33:888-909). Respondents to the Diovan Pediatric Heart Failure Survey confirmed that 'efficacy shown in adult HF trials' and 'Consensus Statements and Guidelines' were the two most important factors they considered when making treatment decisions for pediatric patients with HF (CVAL489K2304 HF Survey, 2011). According to this survey of pediatric cardiologists, current clinical management of pediatric HF includes angiotensin converting enzyme inhibitors (ACEI), angiotensin receptor blockers (ARBs), β-blockers, diuretics, aldosterone-blocking agents, digoxin and anticoagulants.

At this point, no trial has demonstrated an outcome benefit of any pharmacotherapy in children with HF. The largest pediatric HF trial done thus far is the randomized, double-blind, placebo-controlled, parallel-group trial of carvedilol in patients 8 months to 14 years with HF due to congenital heart disease or cardiomyopathy (Shaddy RE, Boucek MM, Hsu DT, et al. (2007) Carvedilol for children and adolescents with heart failure: A randomized controlled trial. JAMA; 298:1171-1179). The primary endpoint for the pediatric carvedilol study was a composite measure of HF outcomes, assessing the response to treatment as worsening, improved or unchanged. While adult HF populations have shown benefit with β-blockade, this study did not meet its composite primary endpoint. This may have been attributable to the known issues related to pediatric HF trials in general including: varying causes of HF, uncertain natural history of HF in children, and trial design challenges unique to the pediatric population.

### Sacubitril and Valsartan (LCZ696)

LCZ696 is a first-in-class angiotensin receptor neprilysin inhibitor (ARNI) being developed for the treatment of cardiovascular diseases such as hypertension and/or heart failure. LCZ696 comprises the anionic forms of sacubitril and valsartan, sodium cations and water molecules in the molar ratio of 1:1:3:2.5, respectively (ratio of 6:6:18:15 in the asymmetric unit cell of the solid state crystal), and which is schematically present in the following formula:

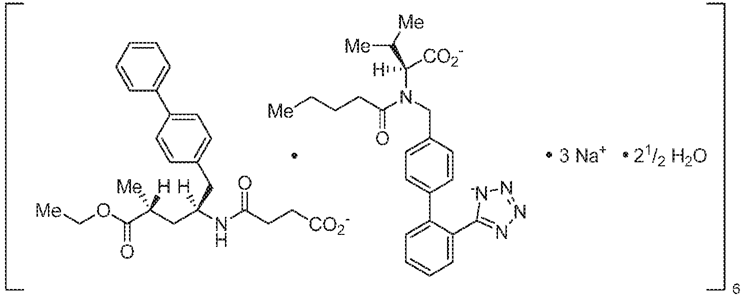

The left molecule depicts the NEP inhibitor prodrug sacubitril (AHU377, (2R,4S)-5-biphenyl-4-yl-4-(3-carboxy-propionylamino)-2-methyl-pentanoic acid ethyl ester, also named *N*-(3-carboxyl-1-oxopropyl)-(4*S*)-(p-phenylphenylmethyl)-4-amino-(2*R*)-methyl butanoic acid ethyl ester; IUPAC name 4-{[(1S,3R)-1-([1,1'-biphenyl]-4-ylmethyl)-4-ethoxy-3-methyl-4-oxobutyl]amino}-4-oxobutanoic acid), whereas the right molecule valsartan, a known angiotensin receptor blocker (ARB).

Ingestion of LCZ696 results in systemic exposure to sacubitril which is converted to the active form LBQ657 (2R,4S)-5-biphenyl-4-yl-4-(3-carboxy-propionyl amino)-2-methyl-pentanoic acid), and valsartan providing inhibition of the angiotensin II type 1 (AT1) receptor, in a 1:1 molar ratio.

Combinations of sacubitril and valsartan, and in particular LCZ696 and formulations thereof, have been previously disclosed in WO 2003/059345, WO 2007/056546, and WO 2009/061713.

"European Medicines Agency decision P/0106/2014 of 5 May 2014" discloses a paediatric investigation plan for a solid unit dosage form for oral administration comprising LCZ696.

### Mode of action:

Neprilysin inhibition leads to enhanced levels of the physiologically active natriuretic peptides (NPs), including atrial natriuretic peptide (ANP). NPs mediate their cardiovascular effects through activation of the natriuretic peptide receptor A (NPR-A) and their second messenger cyclic GMP (cGMP), resulting in potent vasodilation, natriuresis, diuresis, inhibition of the renin angiotensin aldosterone system (RAAS) by reducing renin and aldosterone release, reduced sympathetic drive, and antiproliferative and antihypertrophic effects on vascular endothelium and smooth muscle cells. The angiotensin receptor blocker (ARB) component provides AT1 receptor antagonism, preventing the deleterious effects of angiotensin II and thereby lowering peripheral vascular resistance. By delivering dual and potentially complementary beneficial effects, LCZ696 may offer clinical benefits to patients with cardiovascular and renal disease.

Various uses of combinations of sacubitril and valsartan, and in particular LCZ696, for the treatment of patients with various cardiovascular and/or renal diseases have been described in e.g. WO 2003/059345, WO 2007/056546, WO 2012/027237, WO 2014/029848, WO 2015/030711, and WO 2015/028941.

In particular, neprilysin (NEP) inhibition with chronic oral administration of LCZ696 can promote the endogenous capacity of the body to compensate for Heart Failure (HF) exacerbations by potentiating the activity of natriuretic peptides secreted by the heart in response to cardiac stress and increased intravascular volume. LCZ696, unlike any other therapy for HF, provides concomitant inhibition of NEP and the angiotensin type 1 (AT1) receptor. The resulting increase in natriuretic peptide (NP) activity due to NEP inhibition and AT1 receptor blockade through renin-angiotensin-aldosterone system (RAAS) inhibition have complementary effects on the cardiovascular (CV) system that benefit HF patients.

In PARADIGM-HF (CLCZ696B2314; N=8442), the pivotal Phase 3 study in adult patients with HF with reduced ejection fraction (HFrEF), LCZ696 was superior to enalapril (the standard of care) in delaying time to first occurrence of composite endpoint of CV death or HF hospitalization, with a 20% relative risk reduction (RRR) (p = 0.0000002). In addition, LCZ696 was superior to enalapril in delaying time to CV death with a 20% RRR p=0.00004) and in delaying time to first HF hospitalization with 21% RRR (p=0.00004). PARADIGM-HF also showed that LCZ696 is generally safe and well tolerated in adult patients with HF (McMurray et al, 2014).

### Need:

As set out above, at this point, no trial has demonstrated an outcome benefit of any pharmacotherapy in children with HF. Accordingly, there is a strong need for a new drug for use in children as an alternative to currently available therapies for cardiovascular diseases such as heart failure (HF).

The present invention relates to pharmaceutical compositions comprising a therapeutically effective amount or a prophylactically effective amount of a combination of sacubitril and valsartan in a 1:1 molar ratio for use in the prevention or treatment of heart failure in a human pediatric patient, wherein the human pediatric patient has an age from 1 month to < 18 years, and wherein the patient suffers from chronic heart failure resulting from left ventricular systolic dysfunction, and wherein,
when the patient is from 6 to less than 18 year old, the patient has heart failure of NYHA class II, III or IV, or
when the patient is less than 6 year old, the patient has heart failure of Ross HF classification II-IV, wherein the use comprises the twice daily administration of a single dose of the combination of sacubitril and valsartan in a 1:1 molar ratio from 2 mg / kg body weight to 4 mg / kg body weight of the patient.

Rationale: In both pediatric and adult HF due to systolic dysfunction, there is a decrease in systemic cardiac output. The pathophysiologic adaptation to decreased cardiac output for both adult and pediatric HF involves increased sympathetic tone and activation of the renin-angiotensin system (RAS). In addition, also similar to adult HF, pediatric HF results in increased activation of the natriuretic peptide system. This pathophysiologic neurohumoral activation plays a key role in the progression of HF due to systolic dysfunction in adults and children, and this is why heart failure management in this pediatric HF subset with systemic left ventricular systolic dysfunction is similar to adult HFrEF.

In one embodiment, this invention focuses on a subset of pediatric HF with systemic left ventricular systolic dysfunction which is a more homogeneous pediatric HF population that also has pathophysiology similar to adult HFrEF where LCZ696 has demonstrated a significant mortality and morbidity benefit (McMurray 2014) compared to current standard of care (ACEI).

The efficacy of LCZ696 over the standard of care enalapril for reducing mortality and morbidity in adult HFrEF patients provides strong rationale that LCZ696 has clinically meaningful benefits for pediatric HF patients with reduced left ventricular ejection fraction (LVEF). Despite differences with regards to the etiology of pediatric and adult HF with systolic dysfunction (or reduced LVEF), there is overlap in the pathophysiology and clinical management between both populations, especially for pediatric patients with symptomatic systemic left ventricular systolic dysfunction.

Accordingly, the present disclosure is in a first aspect directed to a pharmaceutical composition comprising a therapeutically effective amount or a prophylactically effective amount of a combination of sacubitril and valsartan in a 1:1 molar ratio for use in the prevention or treatment of heart failure in a human pediatric patient, according to claim 1.

Such combination of sacubitril and valsartan is a pharmaceutical composition comprising a therapeutically effective amount or a prophylactically effective amount of a combination of a 1:1 molar ratio of
(i) valsartan or a pharmaceutically acceptable salt thereof; and
(ii) sacubitril or a pharmaceutically acceptable salt thereof.

In one embodiment thereof, said combination is provided in the form of the compound of the formula (I)

[(A₁)(A₂)](Na⁺)_{y} • x H₂O (I)

wherein
A₁ is valsartan in the anionic form;
A₂ is sacubitril in the anionic form;
Na⁺ is a sodium ion;
y is 1 to 3; and
x is 0 to 3.

In one embodiment thereof, the compound of formula (I) is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl)amino)butyrate] hemipentahydrate (LCZ696).

In one embodiment, the pharmaceutical composition comprises in addition one or more pharmaceutically acceptable carriers.

Further features and advantages of the disclosure will become apparent from the following detailed description of the invention.

### Definitions

Throughout this specification and in the claims that follow, the following terms are defined with the following meanings, unless explicitly stated otherwise. The following definitions may be used independently to provide more specific versions of one or more or (as far as present) all generic terms used above or below, thus defining more specific invention embodiments:
The term "prevention" refers to prophylactic administration to a healthy subject to prevent the development of the conditions mentioned herein. Moreover, the term "prevention" means prophylactic administration to patients being in a pre-stage of the conditions to be treated.

The term "treatment" is understood the management and care of a patient for the purpose of combating the disease, condition or disorder.

The terms "effective amount" or "therapeutically effective amount" refer to an amount of a drug or a therapeutic agent that will elicit the desired biological and/or medical response of a tissue, system or an animal (including man) that is being sought by a researcher or clinician. In particular, the terms "effective amount" or "therapeutically effective amount" refer to the amount of the active ingredient or agent which halts or reduces the progress of the condition being treated or which otherwise completely or partly cures or acts palliatively on the condition, e.g. chronic heart failure.

The terms "patient" include, but are not limited to, humans, dogs, cats, horses, pigs, cows, monkeys, rabbits and mice. The preferred patients are humans.

The terms "administration of" and or "administering a" compound should be understood to mean providing a compound of the invention or a pharmaceutically acceptable salt or ester thereof, or a pro-drug thereof to a subject in need of treatment. The administration of the composition of the present invention in order to practice the present methods of therapy is carried out by administering a therapeutically effective amount of the compounds in the composition to a subject in need of such treatment or prophylaxis. The need for a prophylactic administration according to the methods of the present invention is determined via the use of well-known risk factors. The effective amount of an individual compound is determined, in the final analysis, by the physician in charge of the case, but depends on factors such as the exact disease to be treated, the severity of the disease and other diseases or conditions from which the patient suffers, the chosen route of administration, other drugs and treatments which the patient may concomitantly require, and other factors in the physician's judgment.

The term "prophylactically effective amount" as used herein means the amount of the active compounds in the composition that will elicit the biological or medical response in a tissue, system, subject, or human that is being sought by the researcher, veterinarian, medical doctor or other clinician, to prevent the onset of a disease characterized and / or manifested by atrial enlargement and/or remodeling.

As used herein, the term "about" refers to +/- 20%, +/- 10%, or +/- 5% of a value.

The term "pharmaceutically acceptable", as used herein, refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio.

The term "minitablets" within the scope of this application denotes small tablets with an overall weight of approximately 2 to 30 mg, e.g. approximately 4 to 9 mg, e.g. approximately 7 mg, in their uncoated form, and approximately 2.2 to 32 mg, e.g. approximately 4.1 to 10 mg, e.g. approximately 7.1 to 7.5 mg in their coated form. Minitablets are a specific form of multiparticulates as defined herein. They can be prepared as described herein, including preparation from other, smaller multiparticulates, such as particles, granules or beads. The minitablets may have any shape known to the skilled person for tablets, e.g. round e.g. with a diameter of about 1.25 to 3 mm or as defined elsewhere herein; cylindrical e.g. having a convex upper face and convex lower face and e.g. with a cylindrical diameter and height independently of each other are from 1 to 3 mm or as defined elsewhere herein; or biconvex minitablets e.g. whose height and diameter are approximately equal and are from 1.25 to 3 mm, or as defined elsewhere herein.

The New York Heart Association (NYHA) classification grades the severity of heart failure symptoms as one of four functional classes. The NYHA classification is widely used in clinical practice and in research because it provides a standard description of severity that can be used to assess response to treatment and to guide management. The New York Heart Association functional classification based on severity of symptoms and physical activity:
Class I: No limitation of physical activity. Ordinary physical activity does not cause undue breathlessness, fatigue, or palpitations.
Class II: Slight limitation of physical activity. Comfortable at rest, but ordinary physical activity results in undue breathlessness, fatigue, or palpitations.
Class III: Marked limitation of physical activity. Comfortable at rest, but less than ordinary physical activity results in undue breathlessness, fatigue, or palpitations.
Class IV: Unable to carry on any physical activity without discomfort. Symptoms at rest can be present. If any physical activity is undertaken, discomfort is increased.

Choice of endpoints: Cardiovascular death and heart failure hospitalization both reflect disease-specific endpoints related to progressive worsening of the heart failure syndrome, and experienced by patients with systolic heart failure. These endpoints can be modified by treatments improving this condition, which has generally proved to be the case with drugs such as ACEls, aldosterone antagonists, and β-blockers as well as devices for cardiac resynchronization therapy.

The term "sacubitril and valsartan in a 1:1 molar ratio" as used herein refers to a combination comprising a therapeutically effective amount of a 1:1 molar ratio of
(i) valsartan or a pharmaceutically acceptable salt thereof; and
(ii) sacubitril or a pharmaceutically acceptable salt thereof,

Sacubitril is the INN for N-(3-carboxy-1-oxopropyl)-(4S)-(p-phenylphenylmethyl)-4-amino-2R-methylbutanoic acid ethyl ester. This is a prodrug for (2R,4S)-5-biphenyl-4-yl-4-(3-carboxy-propionyl amino)-2-methyl-pentanoic acid. Sacubitril can be prepared by known methods such as described in U.S. Patent No. 5,217,996.

Valsartan is S-N-valeryl-N-{[2'-(1H-tetrazole-5-yl)-biphenyl-4-yl]-methyl}-valine. Valsartan or (S)-N-valeryl-N-{[2'-(1H-tetrazole-5-yl)-biphenyl-4-yl]-methyl}-valine) or a pharmaceutically acceptable salt thereof that can be purchased from commercial sources or can be prepared according to known methods, such as described in U.S. Patent No. 5,399,578 and EP 0443983.

Valsartan may be used in certain embodiments of the invention in its free acid form, as well as in any suitable salt form.

In one embodiment thereof, the combination comprises a 1:1 molar ratio
(i) of valsartan; and
(ii) of sacubitril or a pharmaceutically acceptable salt thereof, such as sodium or calcium salt.

In another embodiment thereof, said combination is provided in the form of a compound of the formula (I)

[(A₁)(A₂)](Na⁺)_{y} • x H₂O (I)

wherein
A₁ is valsartan in the anionic form;
A₂ is sacubitril in the anionic form;
Na⁺ is a sodium ion;
y is 1 to 3, preferably 1, 2, or 3; and
x is 0 to 3, preferably 0, 0.5, 1, 1.5, 2, 2.5, or 3.

In one embodiment, y is 3 and x is 2.5.

In particular, the compound is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

In a preferred embodiment, the compound trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate is present in crystalline form.

The corresponding active ingredient or a pharmaceutically acceptable salt thereof may also be used in the form of a hydrate or include other solvents used for crystallization.

Preferably, the compounds sacubitril or a salt thereof, valsartan or a salt thereof, or LCZ696 are substantially pure or in a substantially pure form. As used herein, "substantially pure" refers to at least about 90% purity, more preferably at least about 95% and most preferably at least about 98% purity.

Also preferred is that these compounds are solid or a solid form or solid state. The solid, solid form or solid state can be crystalline, partially crystalline, amorphous or poly-amorphous, preferably in the crystalline form.

The term "sacubitril and valsartan in a 1:1 molar ratio" can also refer to alternative complexes or compounds comprising valsartan and sacubitril and linking them together via non-covalent or covalent bonding, optionally via a linker.

### Detailed Description of the Invention

As set out above, at this point, no trial has demonstrated an outcome benefit of any pharmacotherapy in children with HF. Accordingly, there is a strong need for a new drug for use in children as an alternative to currently available therapies for cardiovascular diseases such as heart failure (HF).

The present invention now discloses that the drug LCZ696 (i.e. sacubitril and valsartan in a 1:1 molar ratio) may especially be beneficial on improving or reducing heart failure in pediatric patients, thereby differentiating LCZ696 from currently available cardiovascular drugs for children.

In particular, it is the aim of the present invention to provide a treatment for pediatric heart failure patients, wherein LCZ696 is superior to enalapril for treatment of heart failure as assessed using a global rank endpoint in pediatric HF patients.

And it is an aim of the present invention to provide a treatment for pediatric heart failure patients, wherein LCZ696 is superior to enalapril for reducing the time to first occurrence of the composite of either Category 1 and 2 events (e.g. death and worsening HF).

In another embodiment, it is an aim of the present invention to provide a treatment for pediatric heart failure patients, wherein LCZ696 is superior to enalapril for improving NYHA/Ross functional class.

Accordingly, the present invention relates to the following:
Methods of treatment (not claimed)

The scope of the invention is defined by the claims. Any references in the description to methods of treatment refer to the pharmaceutical compositions of the present invention for use in a method for treatment of the human or animal body by therapy.

Thus, the disclosure encompasses a method for the prevention or treatment of heart failure in a human pediatric patient in need of such prevention or treatment comprising administering to said patient a therapeutically effective amount or a prophylactically effective amount of a combination of sacubitril and valsartan in a 1:1 molar ratio.

In a preferred embodiment, the human pediatric patient has an age from 1 month to < 18 years.

In one embodiment said patient suffers from chronic heart failure resulting from left ventricular systolic dysfunction.

In one embodiment said patient is from 6 to less than 18 year old and has heart failure of NYHA class II, III or IV, or the patient is less than 6 year old and has heart failure of Ross HF classification II-IV.

In another embodiment said patient has a systemic left ventricular ejection fraction (LVEF) of ≤ 40%, preferably ≤ 35, or fractional shortening of ≤ 20%.

In one embodiment thereof, the patient has a Heart Failure etiology selected from Congenital Cardiac Malformation with systemic ventricular systolic dysfunction; Idiopathic Cardiomyopathy; Familial/Inherited and/or Genetic Cardiomyopathy; History of Myocarditis; Neuromuscular Disorder; Inborn Error of Metabolism; Mitochondrial Disorder;

Acquired (Chemotherapy, latrogenic, Infection, Rheumatic, Nutritional); Ischemic (e.g. Kawasaki Disease, post-operative); and Left ventricular non-compaction.

In one embodiment thereof, the patient suffers from chronic heart failure resulting from left ventricular systolic dysfunction and classified as NYHA class II, III or IV, and wherein the patient has a reduced left ventricular ejection fraction (LVEF) of ≤ 40%, preferably ≤ 35%. In another embodiment thereof, the administration reduces the risk of cardiovascular death and hospitalization for heart failure in said patients.

In one embodiment thereof, the therapeutically effective amount or the prophylactically effective amount of a combination of sacubitril and valsartan in a 1:1 molar ratio comprises a daily overall dose of the combination of sacubitril and valsartan in a 1:1 molar ratio from about 10 mg to about 500 mg.

In another embodiment thereof, the therapeutically effective amount or the prophylactically effective amount of a combination of sacubitril and valsartan in a 1:1 molar ratio comprises a daily overall dose of the combination of sacubitril and valsartan in a 1:1 molar ratio from about 4 mg / kg body weight to about 8 mg / kg body weight of the patient, preferably around 6 mg / kg body weight. In one embodiment thereof, the therapeutically effective amount or the prophylactically effective amount of the combination of sacubitril and valsartan in a 1:1 molar ratio comprises a daily overall dose of the combination of sacubitril and valsartan in a 1:1 molar ratio selected from 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, and 8.0 mg / kg body weight of the patient, in particular 6.2 mg / kg body weight.

The combination of sacubitril and valsartan in a 1:1 molar ratio is administered to the patient twice daily.

The therapeutically effective amount or the prophylactically effective amount of a combination of sacubitril and valsartan in a 1:1 molar ratio comprises the twice daily administration of a single dose of the combination of sacubitril and valsartan in a 1:1 molar ratio from about 2 mg / kg body weight to about 4 mg / kg body weight of the patient, preferably around 3 mg / kg body weight. In one embodiment thereof, the therapeutically effective amount or the prophylactically effective amount of the combination of sacubitril and valsartan in a 1:1 molar ratio comprises the twice daily administration of a single dose of the combination of sacubitril and valsartan in a 1:1 molar ratio selected from 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, and 4.0 mg / kg body weight of the patient, in particular 3.1 mg / kg body weight.

In another embodiment thereof, the combination of sacubitril and valsartan in a 1:1 molar ratio is administered to the patient in the form of minitablets each containing 3.125 mg active ingredient (sacubitril and valsartan in a 1:1 molar ratio) per tablet, or in the form of tablets each containing 50 mg, 100 mg or 200 mg ingredient (sacubitril and valsartan in a 1:1 molar ratio) per tablet.

In another embodiment, sacubitril and valsartan in a 1:1 molar ratio are delivered in the form of the compound trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

In one aspect of the present invention which applies to all of the aforementioned treatment options, the pharmaceutical composition is administered to deliver a daily overall dose of the combination of sacubitril and valsartan in a 1:1 molar ratio from about 10 mg to about 500 mg, in particular to about 400 mg.

In one embodiment thereof,
a) the 50 mg dose of sacubitril and valsartan in a 1:1 molar ratio corresponds to 24 mg sacubitril and 26 mg valsartan,
b) the 100 mg dose of sacubitril and valsartan in a 1:1 molar ratio corresponds to 49 mg sacubitril and 51 mg valsartan, and
c) the 200 mg dose of sacubitril and valsartan in a 1:1 molar ratio corresponds to 97 mg sacubitril and 103 mg valsartan.

In another embodiment, sacubitril and valsartan in a 1:1 molar ratio is administered to the patient in the form of minitablets each containing 3.125 mg active ingredient (sacubitril and valsartan in a 1:1 molar ratio) per tablet. The exact dosage to be administered to each patient is determined based on the body weight as set out above, i.e. to accomplish the twice daily administration of a single dose of the combination of sacubitril and valsartan in a 1:1 molar ratio from about 2 mg / kg body weight to about 4 mg / kg body weight of the patient, preferably around 3 mg / kg body weight, in particular 3.1 mg / kg body weight. E.g. for a child of 20 kg, a single dose of 3 mg / kg corresponds to a 60 mg dose of the active ingredient combination to be administered twice daily. A single dose of 3.1 mg / kg corresponds to a 62 mg dose of the active ingredient combination to be administered twice daily.

A 60 mg dose corresponds to about 19 minitablets each containing 3.125 mg active ingredient, or to a 50 mg normal tablet and 3 minitablets.

A 62 mg dose corresponds to about 20 minitablets each containing 3.125 mg active ingredient, or to a 50 mg normal tablet and 4 minitablets.

In one embodiment of the invention the minitablets are administered via capsules, e.g. a capsule can contain a defined number of minitablets. Said number can be selected from 1, 2, 3, 4, 5, 6, 7, 8, 9, 10, 11, 12, 13, 14, 15, 16, 17, 18, 19, and 20; preferably the container, preferably the capsule, contains 4 or 10 minitablets.

In a particular embodiment of the pharmaceutical composition, the combination of sacubitril and valsartan in a 1:1 molar ratio is delivered in the form of the compound trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696), wherein
a) the 50 mg of sacubitril and valsartan in a 1:1 molar ratio corresponds to around 56.6 mg LCZ696,
b) the 100 mg of sacubitril and valsartan in a 1:1 molar ratio corresponds to around 113.1 mg LCZ696,
c) the 200 mg of sacubitril and valsartan in a 1:1 molar ratio corresponds to around 226.2 mg LCZ696, and
d) the 3.125 mg of sacubitril and valsartan in a 1:1 molar ratio corresponds to around 3.534 mg LCZ696.

In one embodiment of the aforementioned ones, the combination of sacubitril and valsartan is delivered in the form of pharmaceutical composition comprising in addition one or more pharmaceutically acceptable carriers.

In another embodiment, said patient is concomitantly receiving standard of care treatment for preventing or reducing risk of experiencing recurrent cardiovascular events.

In a further embodiment, said standard of care treatment comprises treatment with a stable dose of a beta-blocker, an aldosterone antagonist, and/ or a diuretic.

In an alternative embodiment said standard of care treatment comprises treatment with a stable dose of a beta-blocker and optionally an aldosterone antagonist.

### Compounds and compositions for use according to the invention

In a preferred embodiment, the invention encompasses a pharmaceutical composition for use comprising a therapeutically effective amount of trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (Compound LCZ696). Such compounds and pharmaceutical compositions have been previously disclosed in WO2007/056546 and WO 2009/061713.

In one embodiment of the invention for all of its uses, the pharmaceutical composition comprises the the NEP inhibitor pro-drug sacubitril, namely N-(3-carboxy-1-oxopropyl)-(4S)-p-phenylphenylmethyl)-4-amino-(2R)-methylbutanoic acid ethyl ester or the NEP inhibitor N-(3-carboxy-1-oxopropyl)-(4S)-p-phenylphenylmethyl)-4-amino-(2R)-methylbutanoic acid, or pharmaceutically acceptable salts thereof, and the Angiotensin Receptor Blocker valsartan or a pharmaceutically acceptable salt thereof. Such combinations are for example disclosed within international patent application WO 2003/059345.
(i) Valsartan or (S)-N-valeryl-N-{[2'-(1H-tetrazole-5-yl)-biphenyl-4-yl]-methyl}-valine) or a pharmaceutically acceptable salt thereof that can be purchased from commercial sources or can be prepared according to known methods, such as described in U.S. Patent No. 5,399,578 and EP 0443983. Valsartan may be used in certain embodiments of the invention in its free acid form, as well as in any suitable salt form.
(ii) Sacubitril, namely N-(3-carboxy-1-oxopropyl)-(4S)-(p-phenylphenylmethyl)-4-amino-2R-methylbutanoic acid ethyl ester or (2R,4S)-5-biphenyl-4-yl-4(3-carboxy-propionyl amino)-2-methyl-pentanoic acid can be prepared by known methods such as described in U.S. Patent No. 5,217,996.

The pharmaceutical compositions according to the invention can be prepared in a manner known per se and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carriers, especially suitable for enteral or parenteral application.

The pharmaceutical preparations of the invention contain, for example, from about 0.1% to about 100%, e. g. 80% or 90%, or from about 1% to about 60%, of the active ingredient. The term "about" or "approximately", as used herein in each instance, shall have the meaning of within 10%, more preferably within 5%, of a given value or range. Pharmaceutical preparations according to the invention for enteral or parenteral administration are, e.g., those in unit dose forms, such as sugar-coated tablets, tablets, capsules, bars, sachets, granules, syrups, aqueous or oily suspensions or suppositories and furthermore ampoules. These are prepared in a manner known per se, e. g. by means of conventional mixing, granulating, sugar-coating, dissolving or lyophilizing processes. Thus, pharmaceutical preparations for oral use can be obtained by combining the active ingredient with solid carriers, if desired granulating a mixture obtained, and processing the mixture or granules, if desired or necessary, after addition of suitable excipients to give tablets or sugar-coated tablet cores.

Tablets may be formed from the active compound with fillers, for example calcium phosphate; disintegrating agents, for example maize starch, lubricating agents, for example magnesium stearate; binders, for example microcrystalline cellulose or polyvinylpyrrolidone and other optional ingredients known in the art to permit tabletting the mixture by known methods. Similarly, capsules, for example hard or soft gelatin capsules, containing the active compound with or without added excipients, may be prepared by known methods. The contents of the capsule may be formulated using known methods so as to give sustained release of the active compound.

Other dosage forms for oral administration include, for example, aqueous suspensions containing the active compound in an aqueous medium in the presence of a non-toxic suspending agent such as sodium carboxymethylcellulose, and oily suspensions containing the active compounds in a suitable vegetable oil, for example arachis oil.

The active compound may be formulated into granules with or without additional excipients. The granules may be ingested directly by the patient or they may be added to a suitable liquid carrier (e.g. water) before ingestion. The granules may contain disintegrants, e.g. an effervescent pair formed from an acid and a carbonate or bicarbonate salt to facilitate dispersion in the liquid medium.

The dosage of the active ingredient of the composition will, of course, vary with the nature of the severity of the condition to be treated and with the particular compound in the composition and its route of administration. It will also vary according to the age, weight and response of the individual patient.

In the embodiments where the pharmaceutical composition comprises trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate] hemipentahydrate (LCZ696) in the pharmaceutical compositions for use in the context of the present invention, the unit dose of the therapeutic agents sacubitril and valsartan together will be in the range from about 1 to about 1000 mg, such as 40 mg to 400 mg (e.g., 50 mg, 100 mg, 200 mg, 400 mg) per day. Alternatively lower doses may be given, for example doses of 0.5 to 100 mg; 0.5 to 50 mg; or 0.5 to 20 mg per day. As explanatory note, a unit dose of 100 mg LCZ696 delivering 100 mg of the two agents sacubitril and valsartan corresponds to 113.1 mg of trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate]hemipentahydrate. Correspondingly, a unit dose of 50 mg requires 56.6 mg, a unit dose of 200 mg requires 226.2 mg, and a unit dose of 400 mg requires 452.4 mg of trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate]hemipentahydrate, respectively.

Dosages of the sum of the individual compounds sacubitril and valsartan or their respective salts in the combination of the pharmaceutical composition will be in the range from about 1 to about 1000 mg, such as 40 mg to 400 mg and include but are not limited to 5 mg, 20 mg, 25 mg, 40 mg, 50 mg, 80 mg, 100 mg, 200 mg, 400 mg, 800 mg and 1000 mg. Such dosages for the individual compounds sacubitril and valsartan can be considered therapeutically effective amounts or dosage strengths. Ratios for the amount of each compound in the pharmaceutical composition are in the 1:1 molar ratio. Ratios for the amount of each compound in the pharmaceutical composition are in the 1:1 molar ratio to achieve a therapeutic effect. In preferred embodiments, the dosages of the individual compounds sacubitril and valsartan correspond to the same molecular amounts as in a pharmaceutical composition comprising a 50 mg, 100 mg, 200 mg or 400 mg dose of LCZ696. E.g. a 200 mg dose of LCZ696 corresponds approximately to 103 mg valsartan and 97 mg of sacubitril.

In one embodiment of the invention, a solid unit dosage form in form of a minitablet, wherein said minitablet contains an effective amount of the active ingredient is between about 2 mg and about 5 mg per minitablet, particularly from about between 2.5 mg and 4.0 mg per minitablet, corresponding to the respective combined amount of valsartan (free acid) and sacubitril (free acid) in a 1:1 molar ratio. In one preferred embodiment, each minitablet contains an amount of 3.125 mg active ingredient per tablet as just defined.

In one embodiment, the active ingredient is provided in the form of LCZ696. The effective amount of LCZ696 is based on the weight of the two active ingredients sacubitril and valsartan without the weight of the sodium and bound water comprised in the complex; i.e. the effective amount from LCZ696 in the minitablets ranges from about between 2 mg to about 5 mg LCZ696 per unit dosage form, particularly from about between 2.5 mg and 4.0 mg per unit dosage form. In one embodiment of the invention, each minitablet contains an amount as just defined of 3.125 mg LCZ696. Taking the sodium and hydrate water into account, said mini-tablets contain between about 3 mg to about 4 mg, preferably about 3.534 mg, trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate per tablet.

Pharmaceutical compositions as used in the current invention are administered twice (b.i.d.) daily in an immediate release formation or as an extended or sustained release formation. Corresponding doses may be taken, for example, in the morning, at mid-day or in the evening.

The following example is illustrative, but does not serve to limit the scope of the invention described herein.

### Example 1:

Single Dose Study to Evaluate Safety, Tolerability and Pharmacokinetics of LCZ696 Followed by a 52-week Study of LCZ696 Compared With Enalapril in Pediatric Patients With Heart Failure (ClinicalTrials.gov Identifier: NCT02678312).

### STUDY DRUG LCZ696:

LCZ696 refers to the supramolecular complex trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate]hemipentahydrate. This compound and pharmaceutical compositions thereof have been previously disclosed in WO2007/056546 and WO 2009/061713.

LCZ696 is a first-in-class angiotensin receptor neprilysin inhibitor that comprises the molecular moieties of the NEP (neutral endopeptidase EC 3.4.24.11) inhibitor pro-drug AHU377 (N-(3-carboxy-1-oxopropyl)-(4S)-p-phenylphenylmethyl)-4-amino-(2R)-methylbutanoic acid ethyl ester) and the angiotensin receptor blocker valsartan as a single compound. AHU377 is metabolized by enzymatic cleavage to LBQ657 (N-(3-carboxy-1-oxopropyl)-(4S)-p-phenylphenylmethyl)-4-amino-(2R)-methylbutanoic acid), the active inhibitor of neutral endopeptidase, which is the major enzyme responsible for the breakdown of atrial natriuretic peptides.

### Overall Study Design:

This is a multicenter, Open-label, Single Dose Study to Evaluate Safety, Tolerability and Pharmacokinetics of **LCZ696** Followed by a 52-week Randomized, Double-blind, Parallel Group, Active-controlled Study to Evaluate the Efficacy and Safety of **LCZ696** Compared With Enalapril in Pediatric Patients From 1 Month to < 18 Years of Age With Heart Failure Due to Systemic Left Ventricle Systolic Dysfunction.

This study consist of two parts (Part 1 and Part 2). The purpose of Part 1 is to evaluate the way the body absorbs, distributes and removes the drug **LCZ696.** This will help determine the proper dose of **LCZ696** for Part 2 of the study. In particular, in Part 1, the open-label study will determine the PK/PD of single-dose LCZ696 in 36 pediatric HF patients (1 month to <18 years) stratified into 3 age groups (Group 1:6 to <18 years; Group 2:1 to <6 years; Group 3:1 month to <1 year) in a sequential overlapping fashion and in descending age-group order. To ensure that patients are enrolled in both high and low end of the 6 to < 18 years of age group, approximately 50% patients will be enrolled who are 6 to 11 years of age in Group 1.
Group 1 (6 to < 18 years): Patients will be recruited to evaluate PK and PD of LCZ696 analytes following the single dose administration of LCZ696 0.8 mg/kg (N=6 observations) and then the single dose administration of LCZ696 3.1 mg/kg (N=6 observations). (The higher dose for Group 1 (3.1 mg/kg) corresponds to ~200 mg LCZ696 dose in an adult with 65 kg bodyweight. )
Group 2 (1 to < 6 years): Patients will be recruited to evaluate PK and PD of LCZ696 analytes following the single dose administration of LCZ696 0.8 mg/kg (N=6 observations) and then the single dose administration of LCZ696 3.1 mg/kg (N=6 observations).
Group 3 (1 month to < 1 year): Patients will be recruited to evaluate PK and PD of LCZ696 analytes following the single dose administration of LCZ696 0.8 mg/kg (N=6 observations). Since physiologically based pharmacokinetic (PBPK) simulations predict higher exposure in this age group, the higher dose (3.1 mg/kg) will not be evaluated in Group 3.

PK and PD of sacubitril/valsartan analytes will be assessed in each age group following the single dose administration of 0.8 mg/kg (N=6 observations) and then 3.1 mg/kg (N=6 observations) dose (except for Group 3 which only receives the lower dose). The patients who receive the sacubitril/valsartan single dose of 0.8 mg/kg treatment will also have an option to subsequently receive the sacubitril/valsartan 3.1 mg/kg dose. If a patient who received 0.8 mg/kg does not qualify or declines to receive the 3.1 mg/kg dose, an additional patient will be recruited to receive the 3.1 mg/kg dose.

The dose for subsequent Groups will be adjusted based on safety information from previous group(s).

The purpose for Part 2 is to compare the effectiveness and safety of **LCZ696** with enalapril in pediatric heart failure patients over 52 weeks of treatment. In particular, Part 2 is a 52-week randomized, double-blind, parallel-group, active-controlled study. The duration will provide 1-year growth and safety data in growing children. Eligible patients (N = 360) stratified by age and NYHA/Ross classification will be randomized to LCZ696 or enalapril (target dose: 0.2 mg/kg bid; maximum 10 mg bid). A lack of validated efficacy endpoints for these patients led to the development of a novel Global Rank primary endpoint derived by ranking patients (worst to best outcome) based on clinical events, such as death and listing for urgent heart transplant/mechanical life support; worsening HF; and measures of functional assessment (NYHA/Ross) and patient-reported QoL outcomes.

### Objectives:

The purpose of this study is to determine whether pediatric heart failure (HF) patients (1 month to < 18 years old) will derive greater clinical treatment benefit with LCZ696 compared to enalapril over 52 weeks treatment duration. This study includes two parts. Part 1 will determine the dose for Part 2. Part 2 will assess the efficacy and safety of LCZ696 compared to enalapril.

Primary objective(s): (more details below)
Part 1: The primary objective is to determine the pharmacokinetics (PK) and pharmacodynamics (PD) of LCZ696 following single ascending dose administration in pediatric HF patients.
Part 2: The primary objective is to determine whether LCZ696 is superior to enalapril for treatment of heart failure as assessed using a global rank endpoint in pediatric HF patients

### Secondary objectives

Part 2: To determine whether LCZ696 is superior to enalapril for reducing the time to first occurrence of the composite of either Category 1 and 2 events (e.g. death and worsening HF) - see Table 1

**Table 1. Primary endpoint algorithm using ranked analysis**

| Category | Subcategory | Description | Ranking algorithm |
|---|---|---|---|
| 1 | A | Death; UNOS status 1A listing for heart transplant or equivalent; | Rank within this category by time-to-first event All Category 1 events are considered equal |
| | | VAD/ECMO/mechanical ventilation requirement for life support at end of study | |
| 2 | B | Worsening HF with HFH-ICU | Within Category 2, the patients will be ranked first by event subcategory, and subsequently by number of events within each subcategory |
| | C | Worsening HF with hospitalization but without intensive care unit stay (HFH-No ICU) | |
| | | | Further ranking by time-to-first event in the worst subcategory |
| | D | Worsening HF without hospitalization (WHF-No Hosp) | |
| 3 | E | Worsened NYHA/Ross (Table 2) or PGIS based on the last available assessment compared with baseline | Rank by combination of NYHA/Ross and PGIS degree of change |
| | | | Within a group of the same degree of NYHA/Ross and PGIS change, further rank by PedsQL change from baseline |
| 4 | F | Unchanged NYHA/Ross and unchanged PGIS based on the last available assessment compared with baseline | Worst baseline combination of NYHA/Ross functional class and PGIS without change is ranked worse than a better baseline NYHA/Ross functional class and PGIS |
| | | | Within a group of the same baseline NYHA/Ross and PGIS, further rank by PedsQL change from baseline |
| 5 | G | Improved NYHA/Ross or PGIS (neither can be worse) based on the last available assessment compared with baseline | Rank by combination of NYHA/Ross and PGIS degree of change |
| | | | Within a group of the same degree of NYHA/Ross and PGIS change, further rank by PedsQL change from baseline |
| ECMO - extracorporeal membrane oxygenation; HF - heart failure; HFH-ICU - hospitalization with intensive care unit stay; NYHA/Ross - NYHA/Ross class of heart failure; NYHA - New York Heart Association; PedsQL - Pediatric Quality of Life Inventory; PGIS - Patient Global Impression of Severity; UNOS - United Network for Organ Sharing; VAD - ventricular assist device | | | |

Part 2: To determine whether LCZ696 is superior to enalapril for improving NYHA/Ross functional class - see Table 2

**Table 2. NYHA/Modified Ross HF classification for children**

| | | |
|---|---|---|
| | | |

| Class | Modified Ross HF Classification for children | NYHA classification |
|---|---|---|
| I | No limitation or symptoms | No limitation of physical activity. Ordinary physical activity does not cause undue fatigue, palpitation, or dyspnea (shortness of breath) |
| II | No growth failure. Mild tachypnea with feeds in infants and/or mild diaphoresis with feeds in infants and/or dyspnea on exertion in older children | Slight limitation of physical activity. Comfortable at rest. Ordinary physical activity results in fatigue, palpitation, dyspnea, or angina |
| III | Growth failure. Prolonged feeding time in infants. Marked tachypnea | Marked limitation of physical activity. Comfortable at rest. Less than ordinary |
| | with exertion or with feeding. Marked diaphoresis with exertion or with feeding | activity will lead to symptoms |
| IV | Symptoms at rest with tachypnea and/or retractions and/or grunting and/or diaphoresis | Inability to perform any physical activity without discomfort. Symptoms of congestive failure present even at rest; increased discomfort with any physical activity |

Part 2: To determine whether LCZ696 is superior to enalapril for improving the Patient Global Impression of Severity (PGIS) score
Part 2: To characterize the population PK of LCZ696 exposure in pediatric patients with HF
Part 2: To assess the safety and tolerability of LCZ696 compared to enalapril in pediatric patients with HF

### Study Patients

The study population consists of pediatric HF patients 1 month to <18 years, inpatient or outpatient, with systemic left ventricle systolic dysfunction; and includes at least 18 and up to 36 patients in Part 1; and randomizes 360 patients in Part 2 at centers worldwide. For Part 1 and Part 2, the patients will be divided across three groups based on age: Group 1: 6 to <18 years; Group 2: 1 to <6 years; Group 3: 1 month to < 1 year.

| | |
|---|---|
| Ages Eligible for Study: | 1 Month to 17 Years |
| Genders Eligible for Study: | Both |
| Accepts Healthy Volunteers: | No |

### Inclusion Criteria:

- Chronic heart failure resulting from left ventricular systolic dysfunction, and receiving chronic HF therapy (if not newly diagnosed)
- NYHA classification II-IV (older children: 6 to <18 years old) or Ross CHF classification II-IV (younger children: < 6 years old)
- Systemic left ventricular ejection fraction ≤ 40% or fractional shortening ≤20%
- For Part 1 study: patients must be taking the equivalent of at least enalapril 0.2 mg/kg
- Biventricular physiology with systemic left ventricle

### Exclusion Criteria:

- Patient with single ventricle or systemic right ventricle
- Patients listed for heart transplantation (as United Network for Organ Sharing status 1A) or hospitalized waiting for transplant (while on inotropes or with ventricular assist device)
- Sustained or symptomatic ventricular dysrhythmias uncontrolled with drug or device therapy
- Patients within 3 months of corrective cardiovascular surgery or corrective percutaneous intervention for congenital heart/ cardiovascular disease
- Patients with unoperated obstructive or severe regurgitant valvular disease, or significant systemic ventricular outflow obstruction or aortic arch obstruction
- Patients with restrictive or hypertrophic cardiomyopathy
- Active myocarditis
- Renal vascular hypertension
- Moderate-to severe obstructive pulmonary disease
- Serum potassium > 5.3 mmol/L
- History of angioedema
- Allergy or hypersensitivity to ACEI / ARB

### Study Procedures:

| Arms | Assigned Interventions |
|---|---|
| Experimental: Part 1: **LCZ696** open label | Drug: **LCZ696** |
| **LCZ696** open label single dose either 1) 0.8 mg/kg or 2) 3.1 mg/kg or both. After the single dose PK assessment, patients will be maintained on open-label Enalapril or standard of care for heart failure treatment, if patient consents to participate in Part 2. | **LCZ696:** 3.125 mg granules (packaged in capsules containing 4 or 10 granules), 50 mg, 100 mg, 200 mg dosage strengths |
| | Drug: Enalapril |
| | Enalapril will be open label in Part 1 and double blind in Part 2 |
| Active Comparator: Part 2: Enalapril | Drug: Enalapril |
| The target dose for enalapril is 0.2 mg/kg bid (0.4 mg/kg total daily dose) with a maximum dose of 10 mg bid (20 mg total daily dose). | Enalapril will be open label in Part 1 and double blind in Part 2 |
| | Drug: Placebo of **LCZ696** |
| Experimental: Part **2:LCZ696** | Drug: **LCZ696** |
| **LCZ696** 3.125 mg granules and adult formulation (50, 100, 200 mg) can be given based on patient weight. | **LCZ696:** 3.125 mg granules (packaged in capsules containing 4 or 10 granules), 50 mg, 100 mg, 200 mg dosage strengths |
| | Drug: Placebo of Enalapril |

### Details:

### Part 1:

### Primary Outcome Measures:

Pharmacokinetics of LCZ696 analytes (sacubitril, LBQ657, and valsartan):
- Maximum drug concentration in plasma (Cmax) [ Time Frame: 0, 0.5, 1, 2, 4, 8, 10, and optional 24 hours post dosing ]. Cmax will be determined for LCZ696 analytes (sacubitril, LBQ657, and valsartan) by using non-compartmental methods.
- Time to maximum plasma concentration (Tmax) [ Time Frame: 0, 0.5, 1, 2, 4, 8, 10, and optional 24 hours post dosing ]. Tmax will be determined for LCZ696 analytes (sacubitril, LBQ657, and valsartan) by using non-compartmental methods.
- Area under the plasma concentration-time curve from time zero to infinity (AUCinf) and area under the plasma concentration-time curve from time zero to last (AUClast) [ Time Frame: 0, 0.5, 1, 2, 4, 8, 10, and optional 24 hours post dosing ]. AUCinf and AUClast will be determined for LCZ696 analytes (sacubitril, LBQ657, and valsartan) by using non-compartmental methods.
- Clearance from plasma (CL/F) [ Time Frame: 0, 0.5, 1, 2, 4, 8, 10, and optional 24 hours post dosing ]. CL/F will be determined for LCZ696 analytes (sacubitril, LBQ657, and valsartan) by using non-compartmental methods.
- Time required to drug concentration to decrease by half (T 1/2) [ Time Frame: 0, 0.5, 1, 2, 4, 8, 10, and optional 24 hours post dosing ]. T 1/2 will be determined for LCZ696 analytes (sacubitril, LBQ657, and valsartan) by using non-compartmental methods
- Plasma N-terminal pro-brain natriuretic peptide (NTproBNP) [ Time Frame: 0, 4, 8, optional 24 hours post dosing ]. The 24 hour post dose is optional depending on blood volume restrictions.
- Plasma cyclic guanosine monophosphate (cGMP) [ Time Frame: 0, 4, 8, optional 24 hours post dosing ]. The 24 hour post dose is optional depending on blood volume restrictions.
- Urine cGMP [ Time Frame: Between 4 and 8 hours post dose ]. One urine sample at 0 hr (predose) and another urine sample will be collected between 4 to 8 hours post-dose.
- Plasma B-type natriuretic peptide (BNP) [ Time Frame: 0 (pre-dose), 4 and 8 hour post dose ]

### Part 2:

### Primary Outcome Measures:

Percentage of patients falling into each category based on global ranking [ Time Frame: Up to 52 weeks ]
The global ranking is based on clinical events such as death, listing for urgent heart transplant, mechanical life support requirement at end of study, worsening heart failure (HF), New York Heart Association (NYHA)/Ross, Patient Global Impression of Severity (PGIS), Pediatric Quality of Life Inventory (PedsQL) physical functioning domain. The primary endpoint will be derived based on 5 categories ranking worst to best outcome

### Secondary Outcome Measures: •

Time to first occurrence of Category 1 or Category 2 event [ Time Frame: 52 weeks ]: Category 1: Death; United Network for Organ Sharing (UNOS) status 1A listing for heart transplant or equivalent; Ventricular assist device (VAD)/Extracorporeal membrane oxygenation (ECMO)/mechanical ventilation requirement for life support at end of study. Category 2: Worsening HF (WHF); defined by signs and symptoms of WHF that requires an intensification of HF therapy

Change from baseline in NYHA/Ross functional class [ Time Frame: Baseline to 52 weeks] NYHA/Ross functional class will be compared through 52 weeks of double-blind treatment. Change from baseline in Patient Global impression of severity score (PGIS) scale [ Time Frame: Baseline to 52 weeks ]. PGIS scale will be compared for LCZ696 and enalapril through 52 weeks of double-blind treatment

Population PK of LCZ696 analytes (sacubitril, LBQ657, and valsartan): Clearance from plasma in steady state (CL,ss) [ Time Frame: Week 2, 12, 52 ]. The steady state population PK parameter clearance will be estimated to be used in model.

Population PK of LCZ696 analytes (sacubitril, LBQ657, and valsartan): Volume of distribution in steady state [ Time Frame: Week 2, 12, 52 ]. The steady state population PK parameter volume of distribution will be estimated to be used in model.

Population PK of LCZ696 analytes (sacubitril, LBQ657, and valsartan): Absorption rate constant in steady state (Ka,ss) [ Time Frame: Week 2, 12, 52 ] The steady state population PK parameter Ka will be estimated to be used in model.

Population PK of LCZ696 analytes (sacubitril, LBQ657, and valsartan): Time required to drug concentration to decrease by half in steady state (T 1/2,ss) [ Time Frame: Week 2, 12, 52 ] The steady state population PK parameter T 1/2 will be estimated to be used in model.

Population PK of LCZ696 analytes (sacubitril, LBQ657, and valsartan): Maximum drug concentration in plasma at steady state (Cmax,ss) [ Time Frame: Week 2, 12, 52 ] The steady state population PK parameter Cmax will be estimated to be used in model.

Population PK of LCZ696 analytes (sacubitril, LBQ657, and valsartan): Lowest plasma concentration observed during a dosing interval at steady state (Cmin,ss) [ Time Frame: Week 2, 12, 52 ] The steady state population PK parameter Cmin will be estimated to be used in model.

Population PK of LCZ696 analytes (sacubitril, LBQ657, and valsartan): area under the plasma concentration-time curve from time zero to the end of the dosing interval tau at steady state (AUCtau,ss) [ Time Frame: Week 2, 12, 52 ] The steady state population PK parameter AUC will be estimated to be used in model.

## Claims

1. A pharmaceutical composition comprising a therapeutically effective amount or a prophylactically effective amount of a combination of sacubitril and valsartan in a 1:1 molar ratio for use in the prevention or treatment of heart failure in a human pediatric patient, wherein the human pediatric patient has an age from 1 month to < 18 years, and wherein the patient suffers from chronic heart failure resulting from left ventricular systolic dysfunction, and wherein,
when the patient is from 6 to less than 18 year old, the patient has heart failure of NYHA class II, III or IV, or
when the patient is less than 6 year old, the patient has heart failure of Ross HF classification II-IV,
wherein the use comprises the twice daily administration of a single dose of the combination of sacubitril and valsartan in a 1:1 molar ratio from 2 mg / kg body weight to 4 mg / kg body weight of the patient.

2. The pharmaceutical composition for use according to claim 1, wherein the patient has a systemic left ventricular ejection fraction (LVEF) of ≤ 40%, preferably ≤ 35, or fractional shortening of ≤ 20%.

3. The pharmaceutical composition for use according to claim 1 or 2, wherein the patient has a Heart Failure etiology selected from Congenital Cardiac Malformation with systemic ventricular systolic dysfunction; Idiopathic Cardiomyopathy; Familial/Inherited and/or Genetic Cardiomyopathy; History of Myocarditis; Neuromuscular Disorder; Inborn Error of Metabolism; Mitochondrial Disorder; Acquired (Chemotherapy, latrogenic, Infection, Rheumatic, Nutritional); Ischemic (e.g. Kawasaki Disease, post-operative); and Left ventricular non-compaction.

4. The pharmaceutical composition for use according to any one of claims 1 to 3, wherein the patient suffers from chronic heart failure resulting from left ventricular systolic dysfunction and classified as NYHA class II, III or IV, and wherein the patient has a reduced left ventricular ejection fraction (LVEF) of ≤ 40%, preferably ≤ 35%.

5. The pharmaceutical composition for use according to any one of the preceding claims 1 to 4, wherein the administration reduces the risk of cardiovascular death and hospitalization for heart failure in said patients.

6. The pharmaceutical composition for use according to any one of the preceding claims 1 to 5, wherein the combination of sacubitril and valsartan in a 1:1 molar ratio is delivered in the form of the compound trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696).

7. The pharmaceutical composition for use according to any of the preceding claims 1 to 6, wherein the therapeutically effective amount or the prophylactically effective amount of a combination of sacubitril and valsartan in a 1:1 molar ratio comprises a daily overall dose of the combination of sacubitril and valsartan in a 1:1 molar ratio from 10 mg to 500 mg.

8. The pharmaceutical composition for use according to any of the preceding claims 1 to 7, wherein the therapeutically effective amount or the prophylactically effective amount of a combination of sacubitril and valsartan in a 1:1 molar ratio comprises a daily overall dose of the combination of sacubitril and valsartan in a 1:1 molar ratio of 6 mg / kg body weight of the patient.

9. The pharmaceutical composition for use according to any one of the preceding claims 1 to 9, wherein the therapeutically effective amount or the prophylactically effective amount of a combination of sacubitril and valsartan in a 1:1 molar ratio comprises the twice daily administration of a single dose of the combination of sacubitril and valsartan in a 1:1 molar ratio of 3 mg / kg body weight of the patient.

10. The pharmaceutical composition for use according to any one of the preceding claims 1 to 9, wherein the combination of sacubitril and valsartan in a 1:1 molar ratio is administered to the patient in the form of one or more minitablets each containing 3.125 mg active ingredient (sacubitril and valsartan in a 1:1 molar ratio) per tablet, or in the form of tablets each containing 50 mg, 100 mg or 200 mg ingredient (sacubitril and valsartan in a 1:1 molar ratio) per tablet, wherein.
a) the 50 mg dose of sacubitril and valsartan in a 1:1 molar ratio corresponds to 24 mg sacubitril and 26 mg valsartan,
b) the 100 mg dose of sacubitril and valsartan in a 1:1 molar ratio corresponds to 49 mg sacubitril and 51 mg valsartan, and
c) the 200 mg dose of sacubitril and valsartan in a 1:1 molar ratio corresponds to 97 mg sacubitril and 103 mg valsartan.

11. The pharmaceutical composition for use according to claim 10, wherein sacubitril and valsartan in a 1:1 molar ratio are delivered in the form of the compound trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate] hemipentahydrate (LCZ696), and wherein
a) the 50 mg of sacubitril and valsartan in a 1:1 molar ratio corresponds to around 56.6 mg LCZ696,
b) the 100 mg of sacubitril and valsartan in a 1:1 molar ratio corresponds to around 113.1 mg LCZ696,
c) the 200 mg of sacubitril and valsartan in a 1:1 molar ratio corresponds to around 226.2 mg LCZ696, and
d) the 3.125 mg of sacubitril and valsartan in a 1:1 molar ratio corresponds to around 3.534 mg LCZ696.

12. The pharmaceutical composition for use according to any one of the preceding claims 1 to 11, wherein the pharmaceutical composition comprises in addition one or more pharmaceutically acceptable carriers.

13. The pharmaceutical composition for use according to any one of the preceding claims 1 to 12, wherein said patient is concomitantly receiving standard of care treatment for preventing or reducing risk of experiencing recurrent cardiovascular events.

14. The pharmaceutical composition for use according to claim 13, wherein said standard of care treatment comprises treatment with a stable dose of a beta-blocker, an aldosterone antagonist, and/ or a diuretic.

## Patentansprüche

1. Pharmazeutische Zusammensetzung, umfassend eine therapeutisch wirksame Menge oder eine prophylaktisch wirksame Menge einer Kombination von Sacubitril und Valsartan in einem Molverhältnis von 1:1 zur Verwendung bei der Prävention oder Behandlung von Herzinsuffizienz bei einem menschlichen pädiatrischen Patienten, wobei der menschliche pädiatrische Patient ein Alter von 1 Monat bis < 18 Jahren hat und wobei der Patient an chronischer Herzinsuffizienz leidet, die auf eine linksventrikuläre systolische Dysfunktion zurückzuführen ist, und wobei
wenn der Patient zwischen 6 und unter 18 Jahre alt ist, der Patient eine Herzinsuffizienz der NYHA-Klasse II, III oder IV hat, oder
wenn der Patient unter 6 Jahre alt ist, der Patient eine Herzinsuffizienz der Ross-HF-Klassifikation II-IV hat,
wobei die Verwendung die zweimal tägliche Verabreichung einer Einzeldosis der Kombination von Sacubitril und Valsartan in einem Molverhältnis von 1:1 von 2 mg/kg Körpergewicht bis 4 mg/kg Körpergewicht des Patienten umfasst.

2. Pharmazeutische Zusammensetzung zur Verwendung nach Anspruch 1, wobei der Patient eine systemische linksventrikuläre Auswurffraktion (Left Ventricular Ejection Fraction, LVEF) von 40 %, vorzugsweise 35, oder eine Verkürzungsfraktion von 20 % hat.

3. Pharmazeutische Zusammensetzung zur Verwendung nach Anspruch 1 oder 2, wobei der Patient eine Herzinsuffizienzätiologie ausgewählt aus angeborener Herzfehlbildung mit systemischer ventrikulärer systolischer Dysfunktion, idiopathischer Kardiomyopathie, familiärer/erblicher und/oder genetischer Kardiomyopathie, Myokarditis in der Anamnese, neuromuskulärer Störung, angeborenem Stoffwechselfehler, mitochondrialer Störung, erworbener (Chemotherapie, iatrogen, Infektion, rheumatisch, ernährungsbedingt), ischämischer (z. B. Kawasaki-Krankheit, postoperativ) und linksventrikulärer Nicht-Kompaktierung hat.

4. Pharmazeutische Zusammensetzung zur Verwendung nach einem der Ansprüche 1 bis 3, wobei der Patient an einer chronischen Herzinsuffizienz als Folge von linksventrikulärer systolischer Dysfunktion leidet und als NYHA-Klasse II, III oder IV klassifiziert ist und wobei der Patient eine systemische linksventrikuläre Auswurffraktion (Left Ventricular Ejection Fraction, LVEF) von 40 %, vorzugsweise 35 % hat.

5. Pharmazeutische Zusammensetzung zur Verwendung nach einem der vorhergehenden Ansprüche 1 bis 4, wobei durch die Verabreichung das Risiko eines eines kardiovaskulären Todes und einer Krankenhauseinweisung wegen Herzinsuffizienz bei diesen Patienten reduziert wird.

6. Pharmazeutische Zusammensetzung zur Verwendung nach einem der vorhergehenden Ansprüche 1 bis 5, wobei die Kombination von Sacubitril und Valsartan in einem Molverhältnis von 1:1 in Form der Verbindung Trinatrium-[3-((1S,3R)-1-biphenyl-4-ylmethyl-3-ethoxycarbonyl-1-butylcarbamoyl)propionat-(S)-3'-methyl-2'-(pentanoyl-{2"-(tetrazol-5-ylat)biphenyl-4'-ylmethyl}amino)-butyrat]-hemipentahydrat (LCZ696) verabreicht wird.

7. Pharmazeutische Zusammensetzung zur Verwendung nach einem der vorhergehenden Ansprüche 1 bis 6, wobei die therapeutisch wirksame Menge oder prophylaktisch wirksame Menge einer Kombination von Sacubitril und Valsartan in einem Molverhältnis von 1:1 eine Gesamttagesdosis der Kombination von Sacubitril und Valsartan in einem Molverhältnis von 1:1 von 10 mg bis 500 mg umfasst.

8. Pharmazeutische Zusammensetzung zur Verwendung nach einem der vorhergehenden Ansprüche 1 bis 7, wobei die therapeutisch wirksame Menge oder prophylaktisch wirksame Menge einer Kombination von Sacubitril und Valsartan in einem Molverhältnis von 1:1 eine Gesamttagesdosis der Kombination von Sacubitril und Valsartan in einem Molverhältnis von 1:1 von 6 mg/kg Körpergewicht des Patienten umfasst.

9. Pharmazeutische Zusammensetzung zur Verwendung nach einem der vorhergehenden Ansprüche 1 bis 9, wobei die therapeutisch wirksame Menge oder prophylaktisch wirksame Menge einer Kombination von Sacubitril und Valsartan in einem Molverhältnis von 1:1 die zweimal tägliche Verabreichung einer Einzeldosis der Kombination von Sacubitril und Valsartan in einem Molverhältnis von 1:1 von 3 mg/kg Körpergewicht des Patienten umfasst.

10. Pharmazeutische Zusammensetzung zur Verwendung nach einem der vorhergehenden Ansprüche 1 bis 9, wobei die Kombination von Sacubitril und Valsartan in einem Molverhältnis von 1:1 dem Patienten in Form einer oder mehrere Minitabletten mit jeweils 3,125 mg Wirkstoff (Sacubitril und Valsartan in einem Molverhältnis von 1:1) pro Tablette oder in Form von Tabletten mit jeweils 50 mg, 100 mg oder 200 mg Inhaltsstoff (Sacubitril und Valsartan in einem Molverhältnis von 1:1) pro Tablette verabreicht wird, wobei
a) die 50-mg-Dosis von Sacubitril und Valsartan in einem Molverhältnis von 1:1 24 mg Sacubitril und 26 mg Valsartan entspricht,
b) die 100-mg-Dosis von Sacubitril und Valsartan in einem Molverhältnis von 1:1 49 mg Sacubitril und 51 mg Valsartan entspricht und
c) die 200-mg-Dosis von Sacubitril und Valsartan in einem Molverhältnis von 1:1 97 mg Sacubitril und 103 mg Valsartan entspricht.

11. Pharmazeutische Zusammensetzung zur Verwendung nach Anspruch 10, wobei Sacubitril und Valsartan in einem Molverhältnis von 1:1 in Form der Verbindung Trinatrium-[3-((1S,3R)-1-biphenyl-4-ylmethyl-3-ethoxycarbonyl-1-butylcarbamoyl)propionat-(S)-3'-methyl-2'-(pentanoyl-{2"-(tetrazol-5-ylat)biphenyl-4'-ylmethyl}amino)-butyrat]-hemipentahydrat (LCZ696) verabreicht werden und wobei
a) 50 mg Sacubitril und Valsartan in einem Molverhältnis von 1:1 etwa 56,6 mg LCZ696 entsprechen,
b) 100 mg Sacubitril und Valsartan in einem Molverhältnis von 1:1 etwa 113,1 mg LCZ696 entsprechen,
c) 200 mg Sacubitril und Valsartan in einem Molverhältnis von 1:1 etwa 226,2 mg LCZ696 entsprechen und
d) 3,125 mg Sacubitril und Valsartan in einem Molverhältnis von 1:1 etwa 3,534 mg LCZ696 entsprechen.

12. Pharmazeutische Zusammensetzung zur Verwendung nach einem der vorhergehenden Ansprüche 1 bis 11, wobei die pharmazeutische Zusammensetzung darüber hinaus einen oder mehrere pharmazeutisch unbedenkliche Träger umfasst.

13. Pharmazeutische Zusammensetzung zur Verwendung nach einem der vorhergehenden Ansprüche 1 bis 12, wobei der Patient einhergehend eine Standardbehandlung zur Prävention oder Verringerung des Risikos wiederkehrender kardiovaskulärer Ereignisse erhält.

14. Pharmazeutische Zusammensetzung zur Verwendung nach Anspruch 13, wobei die Standardbehandlung die Behandlung mit einer stabilen Dosis eines Betablockers, eines Aldosteronantagonisten und/oder eines Diuretikums umfasst.

## Revendications

1. Composition pharmaceutique comprenant une quantité thérapeutiquement efficace ou une quantité prophylactiquement efficace d'une combinaison de sacubitril et de valsartan dans un rapport molaire de 1:1 destinée à être utilisée dans la prévention ou le traitement de l'insuffisance cardiaque chez un patient pédiatrique humain, le patient pédiatrique humain étant âgé de 1 mois à < 18 ans, et souffrant d'insuffisance cardiaque chronique résultant d'un dysfonctionnement systolique ventriculaire gauche, et où,
lorsque le patient est âgé de 6 à moins de 18 ans, le patient présente une insuffisance cardiaque de classe NYHA II, III ou IV, ou
lorsque le patient est âgé de moins de 6 ans, le patient présente une insuffisance cardiaque de classification Ross HF II à IV,
l'utilisation comprenant l'administration deux fois par jour d'une dose unique de la combinaison de sacubitril et de valsartan dans un rapport molaire de 1:1 de 2 mg/kg de poids corporel à 4 mg/kg de poids corporel du patient.

2. Composition pharmaceutique destinée à être utilisée selon la revendication 1, le patient présentant une fraction d'éjection du ventricule gauche (FEVG) systémique ≤ 40 %, de préférence ≤ 35, ou une fraction de raccourcissement ≤ 20 %.

3. Composition pharmaceutique destinée à être utilisée selon la revendication 1 ou 2, le patient présentant une étiologie d'insuffisance cardiaque choisie parmi une malformation cardiaque congénitale avec dysfonctionnement systolique ventriculaire systémique ; une cardiomyopathie idiopathique ; une cardiomyopathie familiale/héréditaire et/ou génétique ; des antécédents de myocardite ; un trouble neuromusculaire ; une erreur innée du métabolisme ; un trouble mitochondrial ; acquise (chimiothérapie, iatrogène, infection, rhumatismale, nutritionnelle) ; ischémique (par exemple, maladie de Kawasaki, postopératoire) ; et une non-compaction du ventricule gauche.

4. Composition pharmaceutique destinée à être utilisée selon l'une quelconque des revendications 1 à 3, le patient souffrant d'une insuffisance cardiaque chronique résultant d'un dysfonctionnement systolique ventriculaire gauche et classée dans la classe NYHA II, III ou IV, et le patient présentant une fraction d'éjection du ventricule gauche (FEVG) réduite de ≤ 40 %, de préférence ≤ 35 %.

5. Composition pharmaceutique destinée à être utilisée selon l'une quelconque des revendications précédentes 1 à 4, l'administration réduisant le risque de décès cardiovasculaire et d'hospitalisation pour insuffisance cardiaque chez lesdits patients.

6. Composition pharmaceutique destinée à être utilisée selon l'une quelconque des revendications précédentes 1 à 5, dans laquelle la combinaison de sacubitril et de valsartan dans un rapport molaire de 1:1 est délivrée sous la forme du composé [3-((1S,3R)-1-biphényl-4-ylméthyl-3-éthoxycarbonyl-1-butylcarbamoyl)propionate-(S)-3'-méthyl-2'-(pentanoyl{2''-(tétrazol-5-ylate)biphényl-4'-ylméthyl}amino)butyrate] trisodique hémipentahydraté (LCZ696).

7. Composition pharmaceutique destinée à être utilisée selon l'une quelconque des revendications précédentes 1 à 6, la quantité thérapeutiquement efficace ou la quantité prophylactiquement efficace d'une combinaison de sacubitril et de valsartan dans un rapport molaire de 1:1 comprenant une dose totale quotidienne de la combinaison de sacubitril et de valsartan dans un rapport molaire de 1:1 de 10 mg à 500 mg.

8. Composition pharmaceutique destinée à être utilisée selon l'une quelconque des revendications précédentes 1 à 7, la quantité thérapeutiquement efficace ou la quantité prophylactiquement efficace d'une combinaison de sacubitril et de valsartan dans un rapport molaire de 1:1 comprenant une dose totale quotidienne de la combinaison de sacubitril et de valsartan dans un rapport molaire de 1:1 de 6 mg/kg de poids corporel du patient.

9. Composition pharmaceutique destinée à être utilisée selon l'une quelconque des revendications précédentes 1 à 9, la quantité thérapeutiquement efficace ou la quantité prophylactiquement efficace d'une combinaison de sacubitril et de valsartan dans un rapport molaire de 1:1 comprenant l'administration deux fois arrière jour d'une dose unique de la combinaison de sacubitril et de valsartan dans un rapport molaire de 1:1 de 3 mg/kg de poids corporel du patient.

10. Composition pharmaceutique destinée à être utilisée selon l'une quelconque des revendications précédentes 1 à 9, la combinaison de sacubitril et de valsartan dans un rapport molaire de 1:1 étant administrée au patient sous la forme d'un ou plusieurs minicomprimés contenant chacun 3,125 mg de principe actif (sacubitril et valsartan dans un rapport molaire de 1:1) par comprimé, ou sous la forme de comprimés contenant chacun 50 mg, 100 mg ou 200 mg de principe actif (sacubitril et valsartan dans un rapport molaire de 1:1) par comprimé, où
a) la dose de 50 mg de sacubitril et de valsartan dans un rapport molaire de 1:1 correspond à 24 mg de sacubitril et 26 mg de valsartan,
b) la dose de 100 mg de sacubitril et de valsartan dans un rapport molaire de 1:1 correspond à 49 mg de sacubitril et 51 mg de valsartan, et
c) la dose de 200 mg de sacubitril et de valsartan dans un rapport molaire de 1:1 correspond à 97 mg de sacubitril et 103 mg de valsartan.

11. Composition pharmaceutique destinée à être utilisée selon la revendication 10, le sacubitril et le valsartan dans un rapport molaire de 1:1 étant délivrés sous la forme du composé [3-((1S,3R)-1-biphényl-4-ylméthyl-3-éthoxycarbonyl-1-butylcarbamoyl)propionate-(S)-3'-méthyl-2'-(pentanoyl{2''-(tétrazol-5-ylate)biphényl-4'-ylméthyl}amino)butyrate] trisodique hémipentahydraté (LCZ696), et où
a) les 50 mg de sacubitril et de valsartan dans un rapport molaire de 1:1 correspondent à environ 56,6 mg de LCZ696,
b) les 100 mg de sacubitril et de valsartan dans un rapport molaire de 1:1 correspondent à environ 113,1 mg de LCZ696,
c) les 200 mg de sacubitril et de valsartan dans un rapport molaire de 1:1 correspondent à environ 226,2 mg de LCZ696, et
d) les 3,125 mg de sacubitril et de valsartan dans un rapport molaire de 1:1 correspondent à environ 3,534 mg de LCZ696.

12. Composition pharmaceutique destinée à être utilisée selon l'une quelconque des revendications précédentes 1 à 11, la composition pharmaceutique comprenant en outre un ou plusieurs véhicules pharmaceutiquement acceptables.

13. Composition pharmaceutique destinée à être utilisée selon l'une quelconque des revendications précédentes 1 à 12, ledit patient recevant concomitamment un traitement standard pour prévenir ou réduire le risque de subir des événements cardiovasculaires récurrents.

14. Composition pharmaceutique destinée à être utilisée selon la revendication 13, ledit traitement standard comprenant un traitement avec une dose stable d'un bêtabloquant, d'un antagoniste d'aldostérone et/ou d'un diurétique.
